# EUROPEAN PATENT APPLICATION

(11) **EP 2 801 622 A1**
(43) Date of publication of application: **12.11.2014**
(21) Application number: 13167151.3
(22) Date of filing: 08.05.2013
(51) Int. Cl.: C12Q 1/68, C12N 15/63

(54) **Multi-tag reporter system**

(71) Applicant: DeltaCell B.V., 1951 NH Velsen-Noord (NL)
(72) Inventor: The designation of the inventor has not yet been filed
(74) Representative: Jansen, Cornelis Marinus

(57) **Abstract**

The invention provides means and methods for determining the amount and/or activity of at least two, but preferably at least three or more, endogenous compounds within the same cell. Multi-tag reporter systems are provided that enable simultaneous determination of the activity of a large number of cellular endogenous compounds of the same cell.

## Description

The invention relates to the fields of biology, immunology and medicine. More specifically, the invention relates to cellular reporter systems.

Cellular processes often involve a cascade of reactions wherein a signal is transferred between cells or within a cell between cellular compounds. For instance, an extracellular stimulus secreted by one cell in the form of a cytokine can induce an activity in another cell by binding to a cell surface receptor. This binding often induces a conformational change in the cytoplasmic part of the receptor which is transmitted to the intracellular part of the receptor. The signal is further transmitted to other compounds by for instance by post-translational modifications, like the phosphorylation of a second protein. This protein will be altered in its characteristics and acquires new properties. For instance its stability is changed and as a consequence its concentration in the cell is changed, or its enzyme activity is altered, or its binding affinity to other proteins is changed. The post-translational modifications and subsequent altered properties on their turn will induce changes in the properties of other cellular components and proteins. Thus the original signal is typically transmitted to other cellular components by means of changes in post-translational modifications and altered protein properties. Many of these signal transduction steps can occur before the signal arrives in the nucleus. In a typical signal transduction pathway the concentration of a transcription factor within the nucleus is changed. This transcription factor recognizes either another transcription factor or a consensus DNA sequence within a promoter. The change in transcription factor level will affect the expression of one or more genes and thereby resulting in the final effect of the first stimulus. The original stimulus causes thus many changes within a cell. Not only the expression of a gene is altered and thereby the encoded protein level, also other proteins levels may be changed due to post-translational modifications and subsequent increased or decreased protein half-lifes. To understand the effect of a stimulus on signal transduction, it is therefore important to be able to follow as many as possible changes within a signal transduction pathway. Methods that make it possible to follow these signal transduction cascade not only qualitatively but also quantitatively are for instance important for developing new medicines that specifically intervene in a certain pathway.

Cellular processes and pathways are commonly studied in research and development programs. For instance, for drug development the effect of candidate compounds on cellular processes is often studied in order to screen for drugs that counteract a cellular process involved in disease, with as few side effects as possible. Drugs affecting multiple pathways are typically not considered preferred candidates because their beneficial effects are often accompanied by adverse side effects due to their influence on cellular processes that are not related to disease. Therefore, the influence on several cellular processes and pathways in the same cell is preferably studied. *In vitro* assays testing the influence of a compound on one transcription factor or other cellular compound is often not sufficient, because many transcription factors and other cellular compounds are capable of (directly or indirectly) influencing different pathways and//or the expression of various genes, since many cellular compounds are part of multiple signal transduction routes. Therefore, the effect of candidate compounds on cells *in vivo* is preferably measured. In order to measure possible effects on cellular processes or pathways, reporter constructs have been used that contain an inducible promoter sequence operably linked to a reporter gene such as a gene encoding a fluorescent or luminescent protein, in order to visualize a difference in gene expression caused by a certain stimulus. For instance, a cell is provided with a nucleic acid construct containing a promoter that controls the expression of a luciferase gene, wherein the promoter is inducible by a certain transcription factor. Upon providing a stimulus to the cell, it is tested whether luciferase is formed, resulting in a light signal. The light intensity is then indicative for the extent of (indirect) activation of the transcription factor by the stimulus.

It is often desirable to simultaneously study multiple cellular processes or pathways, for instance when a disease is studied, or the effects of candidate drugs. However, with current methods only a limited number of cellular processes or pathways in the same cell can be investigated. For instance, two or more reporter constructs containing an inducible promoter and different reporter genes have been used, but the use of these different constructs is limited, due to the raising difficulties of distinguishing between the signals from the different reporters. Therefore, multiple identical cell lines are often used, each cell line containing one kind of construct, as for instance described in WO 02/052039. However, since many pathways are intertwined and many cellular factors influence multiple different compounds, such separate cell systems often do not provide sufficient information about the overall effects.

There remains, therefore, a need for alternative means and methods for screening cellular processes and/or pathways.

It is an object of the present invention to provide means and methods for determining the amount and/or activity of at least two, but preferably at least three or more, endogenous compounds within the same cell.

It is a further object to provide means and methods for monitoring multiple cellular processes, such as for instance post-translational modifications and the action of proteases.

Accordingly, the present invention provides a method for determining the amount and/or activity of at least two endogenous compounds of a cell, the method comprising:
a) introducing into said cell a first nucleic acid molecule comprising a first promoter sequence, operably linked to a nucleic acid sequence encoding a reporter molecule linked to a first peptide tag, wherein said first promoter sequence has an activity that is inducible by a first endogenous compound of said cell;
b) introducing into said cell a second nucleic acid molecule comprising a second promoter sequence, operably linked to a nucleic acid sequence encoding a reporter molecule linked to a second peptide tag, wherein said second promoter sequence has an activity that is inducible by a second endogenous compound of said cell, and wherein said first peptide tag is different from said second peptide tag;
c) culturing said cell, and optionally providing at least one stimulus of interest to said cell;
d) lysing said cell, thereby producing a cell lysate;
e) contacting at least part of said cell lysate with a first binding compound that is bound to, or present at, a first solid phase, wherein said first binding compound is capable of specifically binding said first peptide tag;
f) contacting at least part of said cell lysate with a second binding compound that is bound to, or present at, a second solid phase, wherein said second binding compound is capable of specifically binding said second peptide tag;
g) removing unbound reporter molecules from said first and second solid phase;
h) determining the amount and/or activity of reporter molecule bound to said first binding compound via said first peptide tag, if present, wherein the amount and/or activity of said reporter molecule linked to said first tag is indicative for the amount and/or activity of said first endogenous compound of said cell, and
i) determining the amount and/or activity of reporter molecule bound to said second binding compound via said second peptide tag, if present, wherein the amount and/or activity of said reporter molecule linked to said second tag is indicative for the amount and/or activity of said second endogenous compound of said cell.

With a method according to the present invention, the amount and/or activity of at least two different endogenous compounds of a cell is determined. Said endogenous compounds are preferably involved in a cellular process such as a signal transduction pathway.

With a method according to the invention, reporter signals are obtained that are indicative for the activity of said endogenous compounds. If a certain circumstance or stimulus results in a higher overall activity, this is often due to a higher expression level of said endogenous compound, resulting in a higher overall activity. In such case, a higher activity is indicative for a higher cellular content of said endogenous compound. A method according to the invention is thus suitable for determining the activity and thereby the amount of endogenous compounds.

Preferably, the amount and/or activity of more than two endogenous compounds is tested. A method according to the invention therefore preferably further comprises:
- introducing into said cell a third nucleic acid molecule comprising a third promoter sequence, operably linked to a nucleic acid sequence encoding a reporter molecule linked to a third peptide tag, wherein said third promoter sequence has an activity that is inducible by a third endogenous compound of said cell, and wherein said third peptide tag is different from said first and second peptide tag;
- contacting at least part of said cell lysate with a third binding compound that is bound to, or present at, a third solid phase, wherein said third binding compound is capable of specifically binding said third peptide tag;
- removing unbound reporter molecules from said third solid phase; and
- determining the amount and/or activity of reporter molecule bound to said third binding compound via said third peptide tag, if present, wherein the amount and/or activity of said third reporter molecule is indicative for the amount and/or activity of said third endogenous compound of said cell.

A major advantage of a method according to the present invention is the large number of individual cellular endogenous compounds that can be tested simultaneously in the same cell. The present inventor has for instance administered 15 different constructs into cells, thereby monitoring the activity of 15 different cellular compounds, without difficulty. A method according to the present invention involves introduction of at least 2, but preferably at least 3, 4, 5, 6, 7, 8, 9 or even at least 10 different nucleic acid constructs into a single cell. In one embodiment the constructs all contain an inducible promoter sequence, operably linked to a reporter gene linked to a peptide tag. The constructs differ from each other in that the reporter genes are linked to sequences encoding different peptide tags. The promoter sequences are preferably all inducible by different cellular compounds. Of note, however, the reporter genes are preferably the same in all constructs (although this is not necessary). Using the same reporter gene for all constructs provides the advantage of facilitating production efficacy, and it provides the possibility to use one preferred reporter which is for instance very sensitive, or readily available. In prior art methods wherein multiple cellular processes are studied, different reporters are used, so that favored reporters often have to be combined with suboptimal reporters in view of limited availability of optimal reporters and the necessity to be capable of distinguishing the reporter signals from each other. With a method according to the invention, the use of suboptimal reporters is not required, even though the amount or activity of many cellular compounds is investigated.

As used herein, a cell can be any cell, be it prokaryotic or eukaryotic. The use of eukaryotic cells is preferred for human drug development. Preferably, cells are used that are capable of being cultured *in vitro.*

An endogenous compound of a cell, also called a cellular endogenous compound, is a compound which is naturally present in said cell, at least under certain cellular circumstances.

A promoter sequence is defined as any sequence capable of initiating transcription of a linked gene.

An inducible promoter sequence is a promoter sequence with a transcription initiation activity that is significantly raised in the presence of an inducer. In the absence of such inducer, the promoter either has a significantly lower basal activity or no detectable activity at all. In a preferred embodiment, said first promoter sequence and/or said second promoter sequence comprises a transcription factor binding site. In another preferred embodiment, said first promoter sequence and/or said second promoter sequence is operably linked to a transcription factor binding site. As used herein, the term "promoter sequence" encompasses promoter sequences containing at least one transcription factor binding site, and promoter sequences that are operably linked to at least one transcription factor binding site.

In one preferred embodiment, a synthetic minimal promoter or a TATA box, operably linked to a transcription factor binding site, is used so that the activity of said minimal promoter is regulated by said transcription factor. Such construct is also referred to as a promoter sequence.

In a particularly preferred embodiment, the activity of said first promoter sequence and/or the activity of said second promoter sequence is inducible by a Wnt factor such as for instance a Wnt protein or catenin. This allows investigation of a Wnt pathway in a cell.

Preferably, a first promoter sequence that is inducible by a first endogenous compound is not inducible by said second endogenous compound. Likewise, a second promoter that is inducible by a second endogenous compound is preferably not inducible by said first endogenous compound. This way, the expression activity of said first promoter sequence is directly proportional to the amount and/or activity of said first endogenous compound in said cell, and the expression activity of said second promoter sequence is directly proportional to the amount and/or activity of said second endogenous compound in said cell. Alternatively, said first promoter sequence that is inducible by a first endogenous compound is also inducible by said second endogenous compound, preferably to a different extent as compared to said second promoter sequence. Such first and second promoter sequences are also useful for determining the amount and/or activity of said second endogenous compound, particularly when the extent of activation of said first and second promoter sequences by said second endogenous compound are known. It is then possible to calculate the amount and/or activity of said second endogenous compound in said cell based on the expression activity of both said first and second promoter sequence.

A constitutively active promoter sequence is a promoter sequence which confers a stable expression level to a linked gene.

A promoter sequence is operably linked to a nucleic acid sequence encoding a reporter molecule linked to a tag if said promoter sequence is capable of initiating transcription of said nucleic acid sequence, at least under specific circumstances such as the presence of an inducer. In one embodiment, an inducible promoter sequence is used that comprises a transcription factor binding site. Alternatively, or additionally, said inducible promoter sequence is operably linked to a transcription factor binding site.

A reporter molecule can be linked to a peptide tag in various ways known in the art. For instance, said reporter molecule is linked to said tag via one or more, but preferably no more than 50, amino acid residues. For instance, a peptide linker with a length of 1-25 amino acid residues is used. In a preferred embodiment, a nucleic acid sequence is used that encodes a fusion protein containing said reporter molecule and said peptide tag.

A reporter molecule may be any detectable molecule known in the art, such as for instance a protein with an enzymatic activity of which the substrate or product or byproduct of the enzymatic activity can be detected. Non-limiting examples of such enzymatic reporter molecules are beta-galactosidase, luciferase, beta-lactamase, peroxidase e.g horseradish-peroxidase, and (alkaline) phosphatase. Preferably, said reporter molecule comprises an enzyme that is capable of catalyzing a light generating reaction, which enables sensitive detection of the emitted light with specially equipped visual instruments such as for instance a luminometer, a CCD camera, or simply by visual detection. In a particularly preferred embodiment, said reporter molecule comprises a luciferase, such as a Firefly or Renilla luciferase. A luciferase is preferred in view of its high sensitivity, allowing the detection of very low expression levels.

A peptide tag is defined herein as an amino acid sequence. In principle, said peptide tag may have any length possible, and even be a full length protein. In a preferred embodiment, said peptide tag has a length of between 4 and 100 amino acids, more preferably between 4 and 50 amino acids, most preferably between 4 and 15 and 30 amino acids. Such peptide tags are particularly suitable for immunological detection methods using binding compounds such as antibodies. As used herein, numerical ranges include the indicated lower and upper limit. Hence, a peptide tag with a length of between 4 and 100 amino acids includes peptide tags with a length of 4 amino acid residues, and peptide tags with a length of 100 amino acid residues.

As used herein, the terms "amino acid" and "amino acid residue" are used interchangeably.

After a cell has been provided with at least two, but preferably at least 3, 4, 5 or more constructs according to the invention, the cell is cultured in order to allow the cellular processes of interest to take place. In one embodiment, at least one stimulus of interest is provided to said cell. Said cell is for instance exposed to a drug, a pathogen, a growth factor, a cytokine, a body fluid such as for instance serum, a biological extract such as for instance a plant extract, a monoclonal or polyclonal antibody, or to other cells or a potentially toxic compound. These stimuli may influence cellular processes, which can be detected with a method according to the invention. After culturing, the cells are lysed, thereby producing a cell lysate, which is a fluid fraction containing cell contents. Methods for lysing cells are well known in the art. Non-limiting examples include mechanical disruption, liquid homogenization, the use of high frequency sound waves, freeze/thaw cycles and manual grinding. Especially when the same reporter gene is present in several, or all, constructs, a reporter signal will probably be present in the cell lysate but at this stage it cannot be established from which constructs the reporter signals originate. Then, at least two, but preferably more, solid phases are used. Each solid phase contains a binding molecule that is specific for one peptide tag. The binding molecule is bound to, or present at, said solid support. For instance, the binding molecules are linked or coated to the solid support, or the binding molecules are present in a solution that is in contact with said solid support, such as for instance a fluid filled well. Preferably, said binding compound is directly or indirectly bound to the solid support because this facilitates the washing away of unbound reporter molecules.

Any solid phase, also called a solid support, known in the art of immunology may be used. The different solid phases may for instance be different wells of an ELISA plate, microparticles such as magnetic beads, agarose beads or sepharose beads, or spatially addressable spots on an array, chip or card, et cetera. In a preferred embodiment, said first and second solid phase are part of an array, microarray or chip.

At least part of the cell lysate is contacted with the binding compounds bound to, or present at, the different solid supports. Since the binding compounds of a first solid support are specific for a first peptide tag, they will only bind a reporter molecule linked to said first peptide tag, if present in the cell lysate. Likewise, a second solid support contains binding compounds that are specific for a second peptide tag. Hence, these binding compounds will only bind a reporter molecule linked to said second peptide tag, if present in the cell lysate. Preferably, said binding compounds are (directly or indirectly) bound to said solid supports. In that case, if the cell lysate contains a reporter molecule linked to a certain peptide tag, the reporter-tag protein will be indirectly bound to said solid support via the peptide tag - binding molecule interaction. Subsequently, unbound reporter molecules are washed away and for each solid support it is determined whether a signal from said reporter molecule is present. Optionally, the amount of reporter molecule is quantified. For instance, when luciferase is used, the emitted light intensity can be determined for each well using for instance a luminometer, a CCD camera, or simply by visual detection. Since different kinds of nucleic acid constructs according to the invention contain a different promoter sequence and a different kind of peptide tag, each peptide tag is indicative for the expression of one promoter sequence only, which, in turn, is indicative for the amount and/or activity of one endogenous cellular compound. For instance, a first promoter sequence is operably linked to a nucleic acid sequence encoding a reporter gene linked to a first peptide tag, whereas a second promoter sequence is operably linked to a nucleic acid sequence encoding a reporter gene linked to a second peptide tag. Said first promoter sequence is inducible by a first endogenous compound, whereas said second promoter sequence is inducible by a second endogenous compound. Hence, only when said first endogenous compound is present or active in said cell, a reporter molecule linked to a first peptide tag will be produced. Likewise, only when said second endogenous compound is present or active in said cell, a reporter molecule linked to a second peptide tag will be produced. If, after incubation with the cell lysate and subsequent removal of unbound reporter molecules, a first solid support containing a binding compound specific for said first peptide tag appears to contain a reporter molecule linked to said first peptide tag, bound to said binding compound, this indicates that said first promoter sequence was induced by said first endogenous compound. The amount and/or activity of a reporter molecule linked to said first peptide tag, present at said first solid support, is thus indicative for, and proportional to, the amount and/or activity of said first endogenous compound of said cell. Likewise, if after incubation with the cell lysate and subsequent removal of unbound reporter molecules a second solid support containing a binding compound specific for said second peptide tag appears to contain a reporter molecule linked to said second peptide tag, bound to said binding compound, this indicates that said second promoter sequence was induced by said second endogenous compound. The amount and/or activity of a reporter molecule linked to said second peptide tag, present at said second solid support, is thus indicative for, and proportional to, the amount and/or activity of said second endogenous compound of said cell. The same holds true for a reporter molecule linked to a third peptide tag. If after incubation with the cell lysate and subsequent removal of unbound reporter molecules a third solid support containing a binding molecule specific for said third peptide tag appears to contain a reporter molecule linked to said third peptide tag, bound to said binding compound, this indicates that said third promoter sequence was induced by said third endogenous compound. The same holds true for a reporter molecule linked to a fourth peptide tag present at a fourth solid support, et cetera.

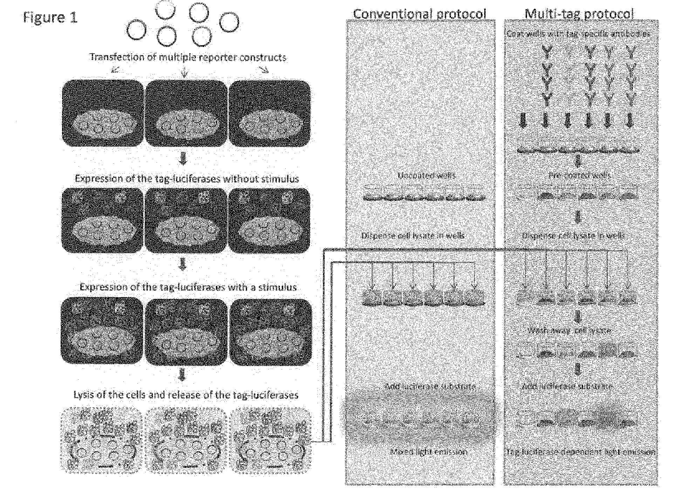

Figure 1 provides a schematic, non-limited overview of one embodiment of a method according to the present invention, as compared to a conventional method.

Rather than an absolute difference between presence and absence of an endogenous compound, a difference between expression levels of endogenous compounds under different circumstances is preferably measured. This is for instance realized by firstly determining a base line level of expression or activity of at least two cellular endogenous compounds with a method according to the present invention, and subsequently performing a method according to the present invention with the same kinds of cells which are cultured under different circumstances. For instance, one batch of cells is cultured without a certain stimulus (such as the presence of a candidate compound or pathogen) whereas another batch of the same kind of cells is cultured with said stimulus. Subsequently, expression levels or activity of at least two cellular endogenous compounds of said first batch of cells without stimulus are compared with the expression levels or activity of the same cellular endogenous compounds of said second batch of cells with stimulus.

Surprisingly, the signals from the reporter molecules bound to said first and second binding compounds present at said first and second solid supports appear to be proportional to the amount and/or activity of said endogenous compounds within said cell, even at high and low cellular concentrations of said endogenous compounds. This was unexpected before the present invention, because the skilled person would expect an overestimate at low expression levels of a cellular endogenous compound, resulting in a low expression level of the reporter-peptide tag protein which expression is inducible by said endogenous compound, and an underestimate at high concentrations of a cellular endogenous compound, resulting in a high expression level of the reporter-peptide tag protein which expression is inducible by said endogenous compound. When a cell lysate containing a low concentration of a certain reporter molecule-peptide tag protein is incubated with binding molecules that are specific for said peptide tag, almost all reporter molecule-peptide tag proteins would be expected to bind the binding compounds, so that a higher percentage of reporter molecule-peptide tag proteins would be expected to bind the binding compounds as compared to a situation wherein a cell lysate with a high concentration of a certain reporter molecule-peptide tag protein is incubated with binding compounds specific for said tag. In the latter case, a relatively lower percentage of reporter molecule-peptide tag proteins would be expected to bind. If this would be the case, the final reporter signal at the first and second solid phases would not be proportional to the original amount and activity of said endogenous compounds in said cells. The present inventor, however, has surprisingly found that also with high or low concentrations of reporter molecule - peptide tag proteins, essentially the same percentage of reporter molecule-peptide tag proteins are bound to the binding compounds. Hence, the same percentage of reporter molecule-peptide tag protein present in the lysate is bound to the binding compounds, irrespective of the absolute concentration of the reporter molecule-peptide tag proteins. Now that the present inventor has provided this insight, it can be concluded that the obtained signals are, indeed, proportional to the amount and activity of the endogenous compounds. Therefore, the methods according to the present invention provide reliable test results which are indicative for the *in vivo* activity of cellular endogenous compounds.

In another aspect of the invention, compositions, kit of parts and vectors are provided for performing a method according to the present invention. Further provided is therefore a composition or a kit of parts or a vector, comprising:
- a first nucleic acid molecule comprising a first promoter sequence, operably linked to a nucleic acid sequence encoding a reporter molecule linked to a first peptide tag, wherein said first promoter sequence has an activity that is inducible by a first cellular endogenous compound; and
- a second nucleic acid molecule comprising a second promoter sequence, operably linked to a nucleic acid sequence encoding a reporter molecule linked to a second peptide tag, wherein said second promoter sequence has an activity that is inducible by a second cellular endogenous compound, and wherein said first peptide tag is different from said second peptide tag. Said composition, kit of parts or vector preferably also comprises a third nucleic acid molecule comprising a third promoter sequence, operably linked to a nucleic acid sequence encoding a reporter molecule linked to a third peptide tag, wherein said third promoter sequence has an activity that is inducible by a third endogenous compound of said cell, and wherein said third peptide tag is different from said first and second peptide tags, so that the activity of at least three endogenous compounds in the same cell can be determined with a method according to the invention.

In yet another embodiment, post-translational modifications of multiple peptide sequences within the same cell is tested with a method according to the invention. Thereto, a nucleic acid molecule is used that comprises a promoter sequence, preferably a constitutively active promoter sequence, operably linked to a nucleic acid sequence encoding a reporter molecule linked to a peptide sequence that can be modified by at least one post-translational modification. Such reporter molecule linked to said peptide sequence is referred to herein as a reporter molecule-peptide protein. Examples of post-translational modifications include, but are not limited to, phosphorylation, dephorphorylation, ubiquitination, glycosylation, palmotination, sumolynation, myristoylation, neddylation, acetylation and breaking of the peptide bond between certain amino acids. Post-translational modifications are often part of a signal-transduction process or represent specifically controlled processes within a cell. Most post-translational modifications are specific for certain proteins or peptide sequences. Normally, the underlying processes of post-translational modifications is difficult to follow. However, with the methods provided by the present invention it has now become possible to test multiple post-translational modifications within a single cell simultaneously, or to screen a cellular post-translational modification process in combination with the activity of one or more other cellular compounds.

When a peptide sequence prone to a post-translational modification is linked to a reporter molecule and used in a method according to the invention, the resulting reporter molecule-peptide protein will be subjected to one or more post-translational modifications under suitable cellular circumstances. Such modification(s) can influence the reporter molecule-peptide protein in several ways. One possible result of such a modification is that the stability or half-life of the reporter molecule-peptide protein is changed. As a consequence, the concentration of the reporter molecule-peptide protein will be different as compared to a situation wherein the reporter molecule-peptide protein has not undergone post-translational modification(s).

In one embodiment of the present invention, post-translational modification(s) resulting in an altered stability of the resulting proteins is tested. At least two nucleic acid molecules are preferably used. A first nucleic acid molecule comprises a promoter sequence, preferably a constitutively active promoter sequence, operably linked to a nucleic acid sequence encoding a reporter molecule that is linked to a first peptide sequence which is, in principle, prone to one or more post-translational modifications. A second nucleic acid molecule comprises a promoter sequence, preferably a constitutively active promoter sequence, operably linked to a nucleic acid sequence encoding a reporter molecule that is linked to a second peptide sequence which is, in principle, prone to one or more post-translational modifications. Said promoter sequences, and said nucleic acid sequences encoding a reporter molecule, of the two different nucleic acid molecules are preferably the same, but the first and second peptide sequences prone to one or more post-translational modifications differ from each other. As used herein, "prone to one or more post-translational modifications" means that said peptide can, in principle, be subjected to one or more cellular post-translational modification reactions, at least under certain cellular circumstances. Throughout this application, the term "prone to one or more post-translational modifications" also encompasses candidate peptides which are tested for their capability of becoming post-translationally modified.

In one embodiment, said first and second peptide sequences prone to one or more post-translational modifications also function as the "peptide tag" in a method according to the present invention. The function of the peptide tag is explained in detail herein before. Alternatively, said first and/or second nucleic acid molecules contain an additional sequence encoding a peptide tag, besides the reporter gene and the sequence encoding the peptide prone to post-translational modification(s).

After a cell has been provided with at least two, but preferably at least 3, 4, 5 or more constructs according to the present invention the cell is cultured in order to allow the cellular processes of interest to take place. In one embodiment, at least one stimulus of interest is provided to said cell in order to investigate its capability of influencing post-translational modification(s).

After culturing, the cells are lysed, thereby producing a cell lysate. When the same reporter gene was present in several, or all, nucleic acid molecules, a reporter signal will probably be present in the cell lysate but at this stage it cannot be established from which constructs the reporter signals originate. Then, at least two, but preferably more, solid phases are used. Each solid phase contains a binding compound that is specific for a peptide tag. A first solid phase contains binding compounds specific for said first peptide tag and a second solid phase contains binding compounds specific for said second peptide tag. As explained above, in this embodiment the first peptide tag is either the first post-translational modification-prone peptide itself, or an additional peptide tag linked to the reporter molecule and the post-translational modification-prone peptide. For instance, a binding compound, such as for instance an antibody or a functional part or functional equivalent thereof, that is used to capture the tag can be chosen in such a way that it recognizes the post-translational modification prone peptide only when the peptide sequence is actually post-translationally modified. Many antibodies have been developed that only recognize such modified sequences. For instance, phospho-specific antibodies are known to recognize certain peptide sequences only when specific amino acids in this sequence are phosphorylated. When these sequences are linked to the reporter molecule then capture of this reporter molecule is only possible when the peptide tag is phosphorylated. The addition of a separate peptide tag can then be avoided.

As explained herein before, the binding compounds are bound to, or present at, said solid supports. For instance, the binding compounds are linked or coated to the solid supports, or the binding compounds are present in a solution that is in contact with said solid support, such as for instance a fluid filled well. Preferably, said binding compounds are directly or indirectly bound to the solid supports because this facilitates the washing away of unbound reporter molecules.

At least part of the cell lysate is contacted with the binding compounds bound to, or present at, the different solid supports. Since the binding compounds of a first solid support are specific for a first peptide tag, they will only bind a reporter molecule linked to said first peptide tag (be it the post-translationally modified peptide itself or a separate peptide tag linked to the reporter molecule-peptide protein), if present in the cell lysate. Likewise, a second solid support contains binding compounds that are specific for a second peptide tag. Hence, these binding compounds will only bind a reporter molecule linked to said second peptide tag (be it the post-translationally modified peptide itself or a separate peptide tag linked to the reporter molecule-peptide protein), if present in the cell lysate. Preferably, said binding compounds are (directly or indirectly) bound to a solid support. In that case, if the cell lysate contains a reporter molecule linked to a certain peptide tag (the post-translationally modified peptide itself or a separate peptide tag), the reporter-peptide tag construct will be indirectly bound to said solid support via the peptide tag - binding compound interaction. Subsequently, unbound reporter molecules are washed away and for each solid support it is determined whether a reporter molecule signal is present. Preferably, the amount of reporter molecule is quantified. For instance, when luciferase is used as reporter molecule, the emitted light intensity can be determined for each well using for instance a luminometer, a CCD camera, or simply by visual detection. When post-translational modifications resulting in an altered stability of the resulting protein are tested, the amount of reporter molecule at a solid support is proportional to the extent of post-translational modification(s) of said peptide prone to post-translational modification(s). Hence, a cell's possibility of post-translationally modifying multiple peptide sequences, as well as the influence of certain stimuli or circumstances, can now be tested in the same cell simultaneously.

Thus, when a reporter molecule linked to a post-translational modification prone peptide is expressed by a promoter and transfected within a cell, the concentration of the reporter molecule-peptide protein, which is post-translationally modifiable, is dependent upon the extent of post-translational modification that takes place in a cell. When a single reporter molecule- post-translational modifiable peptide protein is present in a cell or cell-lysate, the activity of the cellular post-translational modification process is reflected by the reporter molecule signal alone. However, when more than one construct according to the invention is present, or when such a construct is co-expressed with a conventional gene expression reporter, the different reporter molecule signals can be distinguished from each other in a method of the invention by the peptide tag (be it the post-translationally modified peptide itself or a separate peptide tag linked to the reporter molecule-peptide protein) that is linked to the reporter molecule. In this way it is possible to express different, post-translationally modifiable, reporter molecule-peptide proteins, each of them for instance representing a different biological process. It is therefore now for the first time possible to follow a large number of different biological processes within one population of cells simultaneously.

Further provided is therefore a method for testing post-translational modifications of at least two peptide sequences within one cell, the method comprising:
a) introducing into said cell a first nucleic acid molecule comprising a promoter sequence, operably linked to a nucleic acid sequence encoding a reporter molecule linked to a first peptide that is prone to one or more post-translational modifications, said nucleic acid sequence optionally also encoding a first peptide tag;
b) introducing into said cell a second nucleic acid molecule comprising a promoter sequence, operably linked to a nucleic acid sequence encoding a reporter molecule linked to a second peptide that is prone to one or more post-translational modifications, said nucleic acid sequence optionally also encoding a second peptide tag, wherein said first peptide that is prone to one or more post-translational modifications is different from said second peptide that is prone to one or more post-translational modifications, and, optionally, wherein said first peptide tag is different from said second peptide tag;
c) culturing said cell, and optionally providing at least one stimulus of interest to said cell;
d) lysing said cell, thereby producing a cell lysate;
e) contacting at least part of said cell lysate with a first binding compound that is bound to, or present at, a first solid phase, wherein said first binding compound is capable of specifically binding said first peptide that is prone to one or more post-translational modifications, or said first peptide tag;
f) contacting at least part of said cell lysate with a second binding compound that is bound to, or present at, a second solid phase, wherein said second binding compound is capable of specifically binding said second peptide that is prone to one or more post-translational modifications, or said second peptide tag;
g) removing unbound reporter molecules from said first and second solid phase;
h) determining the amount and/or activity of reporter molecule (if present) bound to said first binding compound via said first peptide that is prone to one or more post translational modifications, or via said first peptide tag, wherein the amount and/or activity of said reporter molecule is proportional to the extent of post translational modification of said first peptide that is prone to one or more post translational modifications, and
i) determining the amount and/or activity of reporter molecule (if present) bound to said second binding compound via said second peptide that is prone to one or more post-translational modifications or via said second peptide tag, wherein the amount and/or activity of said reporter molecule is proportional to the extent of post-translational modification of said second peptide that is prone to one or more post-translational modifications.

Preferably, said post-translational modifications are associated with the stability or amount of the resulting modified protein, so that the cellular concentration of a certain protein is proportional to the extent of post-translational modification of said protein. In this case, any binding compound can be used that is either capable of specifically binding the modified peptide sequence, and/or the unmodified peptide sequence, and/or the peptide tag, since the amount of protein that contains said peptide prone to post-translational modification(s) is in this embodiment proportional to the extent of post-translational modification(s) that has occurred in the cell.

In one preferred embodiment, a binding compound is used that is only capable of specifically binding said peptide prone to post-translational modification(s) when it is actually modified. A non-limiting example of such binding compound is a phosphor-specific antibody which only recognizes certain peptide sequences when specific amino acids are phosphorylated. In this embodiment, post-translationally modified peptides are directly detected. Therefore, this embodiment is also particularly suitable for measuring post-translational modifications that are not associated with the stability and amount of the resulting modified protein.

In a preferred embodiment, post-translational modifications of more than two peptide sequences within a single cell is tested. A method according to the invention therefore preferably further comprises:
- introducing into said cell a third nucleic acid molecule comprising a promoter sequence, operably linked to a nucleic acid sequence encoding a reporter molecule linked to a third peptide that is prone to one or more post-translational modifications, said nucleic acid sequence optionally also encoding a third tag, wherein said third peptide that is prone to one or more post-translational modifications is different from said first and second peptides that are prone to one or more post-translational modifications, and, optionally, wherein said third peptide tag is different from said first and second peptide tags;
- contacting at least part of said cell lysate with a third binding compound that is bound to, or present at, a third solid phase, wherein said third binding compound is capable of specifically binding said third peptide that is prone to one or more post-translational modifications, or said third peptide tag;
- removing unbound reporter molecules from said third solid phase; and
- determining the amount and/or activity of reporter molecule (if present) bound to said third binding compound via said third peptide that is prone to one or more post-translational modifications, or via said third peptide tag, wherein the amount and/or activity of said reporter molecule is proportional to the extent of post translational modification of said third peptide that is prone to one or more post translational modifications.

In another aspect of the invention, compositions, kit of parts and vectors are provided for performing a method according to the present invention for testing post-translational modifications of at least two peptide sequences within one cell. Further provided is therefore a composition or a kit of parts or a vector, comprising:
- a first nucleic acid molecule comprising a promoter sequence, operably linked to a nucleic acid sequence encoding a reporter molecule linked to a first peptide that is prone to one or more post-translational modifications, said nucleic acid sequence optionally also encoding a first peptide tag; and
- a second nucleic acid molecule comprising a promoter sequence, operably linked to a nucleic acid sequence encoding a reporter molecule linked to a second peptide that is prone to one or more post-translational modifications, said nucleic acid sequence optionally also encoding a second peptide tag, wherein said first peptide that is prone to one or more post-translational modifications is different from said second peptide that is prone to one or more post-translational modifications, and, optionally, wherein said first peptide tag is different from said second peptide tag.

Said composition or kit of parts or vector preferably also comprises a third nucleic acid molecule comprising a promoter sequence, operably linked to a nucleic acid sequence encoding a reporter molecule linked to a third peptide that is prone to one or more post-translational modifications, said nucleic acid sequence optionally also encoding a third peptide tag, wherein said third peptide that is prone to one or more post-translational modifications is different from said first and second peptides that are prone to one or more post-translational modifications, and, optionally, wherein said third peptide tag is different from said first and second peptide tags, so that post-translational modifications of at least three peptide sequences within the same cell can be determined with a method according to the invention.

A method according to the present invention is also particularly suitable for determining the activity of one or more proteases in a cell, such as for instance a caspase. To this end, a nucleic acid molecule is used that comprises a promoter sequence, preferably a constitutively active promoter, operably linked to a nucleic acid sequence encoding a reporter molecule which is linked to a peptide tag via a peptide sequence that is a target for degradation by a protease. For instance, the target sequence of caspase is Aspartic acid - Glutamic acid - Valine - Aspartic acid (written as DEVD using the amino acid one letter code). If a nucleic acid sequence encoding this DEVD target sequence is inserted between a sequence encoding a reporter protein and a sequence encoding a peptide tag, expression of such construct will yield a reporter - DEVD - peptide tag fusion protein. If caspase is active within the cell, the DEVD sequence will be cleaved, resulting in a separation of the peptide tag from the reporter protein. Subsequently, when the cell lysate is administered to a solid phase containing a binding compound specific for said peptide tag, the tag will be bound but the reporter protein will not, since it is cleaved from the peptide tag. As a result, no reporter signal will be present after the washing step, or the reporter signal will be significantly lower, as compared to a situation wherein caspase is less active in the cell. In this embodiment, therefore, a reporter protein signal is inversely proportional to the protease activity. It is possible to use multiple constructs with different protease cleavage sites in order to determine the activity of several proteases inside a cell. Further provided is therefore a method for determining the activity of at least two different proteases in a cell, the method comprising:
a) introducing into said cell a first nucleic acid molecule that comprises a promoter sequence, preferably a constitutively active promoter, operably linked to a nucleic acid sequence encoding a reporter molecule which is linked to a first peptide tag via a first peptide sequence that is a target for degradation by a first protease;
b) introducing into said cell a second nucleic acid molecule that comprises a promoter sequence, preferably a constitutively active promoter, operably linked to a nucleic acid sequence encoding a reporter molecule which is linked to a second peptide tag via a second peptide sequence that is a target for degradation by a second protease, wherein said first peptide sequence that is a target for degradation by a protease is different from said second peptide sequence that is a target for degradation by a protease, and wherein said first peptide tag is different from said second peptide tag;
c) culturing said cell, and optionally providing at least one stimulus of interest to said cell;
d) lysing said cell, thereby producing a cell lysate;
e) contacting at least part of said cell lysate with a first binding compound that is bound to, or present at, a first solid phase, wherein said first binding compound is capable of specifically binding said first peptide tag;
f) contacting at least part of said cell lysate with a second binding compound that is bound to, or present at, a second solid phase, wherein said second binding compound is capable of specifically binding said second peptide tag;
g) removing unbound reporter molecules from said first and second solid phase;
h) determining the amount and/or activity of reporter molecule bound to said first binding compound via said first peptide tag, if present, wherein the amount and/or activity of said reporter molecule is inversely proportional to the activity of said first protease, and
i) determining the amount and/or activity of reporter molecule bound to said second binding compound via said second peptide tag, if present, wherein the amount and/or activity of said reporter molecule is inversely proportional to the activity of said second protease.

Preferably, the activity of more than two proteases within a single cell is tested. A method according to the invention therefore preferably further comprises:
- introducing into said cell a third nucleic acid molecule that comprises a promoter sequence, preferably a constitutively active promoter, operably linked to a nucleic acid sequence encoding a reporter molecule which is linked to a third peptide tag via a third peptide sequence that is a target for degradation by a third protease, wherein said third peptide sequence that is a target for degradation by a third protease is different from said first and second peptide sequences that are a target for degradation by a protease, and wherein said third peptide tag is different from said first and second peptide tags;
- contacting at least part of said cell lysate with a third binding compound that is bound to, or present at, a third solid phase, wherein said third binding compound is capable of specifically binding said third peptide tag;
- removing unbound reporter molecules from said third solid phase; and
- determining the amount and/or activity of reporter molecule bound to said third binding compound via said third peptide tag, if present, wherein the amount and/or activity of said reporter molecule is inversely proportional to the activity of said third protease in said cell.

In another aspect of the invention, compositions, kit of parts and vectors are provided for performing a method according to the present invention for testing the activity of at least two different proteases of a cell. Further provided is therefore a composition or a kit of parts or a vector, comprising:
- a first nucleic acid molecule that comprises a promoter sequence, preferably a constitutively active promoter, operably linked to a nucleic acid sequence encoding a reporter molecule which is linked to a first peptide tag via a first peptide sequence that is a target for degradation by a first protease; and
- a second nucleic acid molecule that comprises a promoter sequence, preferably a constitutively active promoter, operably linked to a nucleic acid sequence encoding a reporter molecule which is linked to a second peptide tag via a second peptide sequence that is a target for degradation by a second protease, wherein said first peptide sequence that is a target for degradation by a protease is different from said second peptide sequence that is a target for degradation by a protease, and wherein said first peptide tag is different from said second peptide tag.

Said composition or kit of parts or vector preferably also comprises a third nucleic acid molecule that comprises a promoter sequence, preferably a constitutively active promoter, operably linked to a nucleic acid sequence encoding a reporter molecule which is linked to a third peptide tag via a third peptide sequence that is a target for degradation by a third protease, wherein said third peptide sequence that is a target for degradation by a third protease is different from said first and second peptide sequences that are a target for degradation by a protease, and wherein said third peptide tag is different from said first and second peptide tags, so that the activity of at least three different proteases in a cell can be determined with a method according to the invention.

It is, of course, also possible to combine a construct according to the invention containing a protease cleavage site, as described above, with any construct described herein before. For instance, it is possible to simultaneously investigate the amount and/or activity of a protein involved in a signal transduction pathway and the activity of a protease within a single cell with a method according to the invention. Such method is performed using a first nucleic acid molecule comprising a first promoter sequence, operably linked to a nucleic acid sequence encoding a reporter molecule linked to a first peptide tag, wherein said first promoter sequence has an activity that is inducible by said protein involved in a signal transduction pathway, and using a second nucleic acid molecule comprising a second promoter sequence, preferably a constitutively active promoter, operably linked to a nucleic acid sequence encoding a reporter molecule which is linked to a second peptide tag via a peptide sequence that is a target for degradation by a protease, wherein said first peptide tag is different from said second peptide tag. Both nucleic acid molecules are introduced into a cell and after performing the reaction steps according to the present invention the reporter signal at a first and second solid support is measured. In this particular embodiment, the reporter signal present at said first solid support is proportional to the amount and/or activity of said protein involved in a signal transduction pathway, whereas the reporter signal present at said second solid support is inversely proportional to the activity of said protease.

Further provided is therefore a method for determining the amount and/or activity of at least one endogenous compound of a cell and the activity of at least one protease of said cell, the method comprising:
a) introducing into said cell a first nucleic acid molecule comprising a first promoter sequence, operably linked to a nucleic acid sequence encoding a reporter molecule linked to a first peptide tag, wherein said first promoter sequence has an activity that is inducible by a first endogenous compound of said cell;
b) introducing into said cell a second nucleic acid molecule that comprises a promoter sequence, preferably a constitutively active promoter, operably linked to a nucleic acid sequence encoding a reporter molecule which is linked to a second peptide tag via a peptide sequence that is a target for degradation by a protease, wherein said first peptide tag is different from said second peptide tag;
c) culturing said cell, and optionally providing at least one stimulus of interest to said cell;
d) lysing said cell, thereby producing a cell lysate;
e) contacting at least part of said cell lysate with a first binding compound that is bound to, or present at, a first solid phase, wherein said first binding compound is capable of specifically binding said first peptide tag;
f) contacting at least part of said cell lysate with a second binding compound that is bound to, or present at, a second solid phase, wherein said second binding compound is capable of specifically binding said second peptide tag;
g) removing unbound reporter molecules from said first and second solid phase;
h) determining the amount and/or activity of said reporter molecule bound to said first binding compound via said first peptide tag, wherein the amount and/or activity of said reporter molecule is proportional to the amount and/or activity of said first endogenous compound of said cell, and
i) determining the amount and/or activity of said reporter molecule bound to said second binding compound via said second peptide tag, wherein the amount and/or activity of said reporter molecule is inversely proportional to the activity of said second protease.

In another aspect of the invention, compositions, kit of parts and vectors are provided for performing a method according to the present invention for determining the amount and/or activity of at least one endogenous compound of a cell and the activity of at least one protease of said cell. Further provided is therefore a composition or a kit of parts or a vector, comprising:
- a first nucleic acid molecule comprising a first promoter sequence, operably linked to a nucleic acid sequence encoding a reporter molecule linked to a first peptide tag, wherein said first promoter sequence has an activity that is inducible by a first endogenous compound of said cell; and
- a second nucleic acid molecule that comprises a second promoter sequence, preferably a constitutively active promoter, operably linked to a nucleic acid sequence encoding a reporter molecule which is linked to a second peptide tag via a peptide sequence that is a target for degradation by a protease, wherein said first peptide tag is different from said second peptide tag.

Said composition or kit of parts or vector preferably also comprises a third nucleic acid molecule as described herein before. In one embodiment said third nucleic acid molecule comprises a promoter sequence, preferably a constitutively active promoter, operably linked to a nucleic acid sequence encoding a reporter molecule which is linked to a third peptide tag via a second peptide sequence that is a target for degradation by a second protease, wherein said second peptide sequence that is a target for degradation by a second protease is different from said first peptide sequence that is a target for degradation by a first protease, and wherein said third peptide tag is different from said first and second peptide tags, so that the amount and/or activity of at least one endogenous compound and the activity of at least two different proteases in a cell can be determined with a method according to the invention.

In one embodiment said third nucleic acid molecule comprises a promoter sequence, operably linked to a nucleic acid sequence encoding a reporter molecule linked to a third peptide tag, wherein said promoter sequence has an activity that is inducible by a second endogenous compound of said cell, wherein said second endogenous compound is different from said first endogenous compound and wherein said third peptide tag is different from said first and second peptide tags, so that the amount and/or activity of at least two endogenous compounds of a cell and the activity of at least one different protease in said cell can be determined with a method according to the invention.

In yet another embodiment, the amount and/or activity of a protein involved in a signal transduction pathway and the post-translational modification of a peptide sequence within one cell is determined simultaneously using a method according to the present invention, using a first nucleic acid molecule comprising a first promoter sequence, operably linked to a nucleic acid sequence encoding a reporter molecule linked to a first peptide tag, wherein said first promoter sequence has an activity that is inducible by said protein involved in a signal transduction pathway, and a second nucleic acid molecule comprising a promoter sequence, preferably a constitutively active promoter, operably linked to a nucleic acid sequence encoding a reporter molecule linked to a peptide that is prone to one or more post-translational modifications, said nucleic acid sequence optionally also encoding a second peptide tag. If said second peptide tag is present, it is different from said first peptide tag in order to distinguish the reporter proteins bound to the first peptide tag from reporter molecules bound to the second peptide tag, using binding compounds that are specific for said first peptide tag and other binding compounds specific for said second peptide tag, as described herein before. Using a method according to the present invention, both nucleic acid molecules are introduced into a cell and after performing the reaction steps according to the present invention the reporter signal, if any, is measured at a first and second solid support. In this particular example, the reporter signal present at said first solid support is proportional to the amount and/or activity of said protein involved in a signal transduction pathway, whereas the reporter signal present at said second solid support is proportional to the post-translational modification of said peptide sequence.

Further provided is therefore a method for determining the amount and/or activity of at least one endogenous compound of a cell and for testing post-translational modification of at least one peptide sequence within said cell, the method comprising:
a) introducing into said cell a first nucleic acid molecule comprising a first promoter sequence, operably linked to a nucleic acid sequence encoding a reporter molecule linked to a first peptide tag, wherein said first promoter sequence has an activity that is inducible by a first endogenous compound of said cell;
b) introducing into said cell a second nucleic acid molecule comprising a promoter sequence, operably linked to a nucleic acid sequence encoding a reporter molecule linked to a peptide that is prone to one or more post-translational modifications, said nucleic acid sequence optionally also encoding a second peptide tag, wherein said second peptide tag, if present, is different from said first peptide tag;
c) culturing said cell, and optionally providing at least one stimulus of interest to said cell;
d) lysing said cell, thereby producing a cell lysate;
e) contacting at least part of said cell lysate with a first binding compound that is bound to, or present at, a first solid phase, wherein said first binding compound is capable of specifically binding said first peptide tag;
f) contacting at least part of said cell lysate with a second binding compound that is bound to, or present at, a second solid phase, wherein said second binding compound is capable of specifically binding said peptide that is prone to one or more post-translational modifications, or said second peptide tag;
g) removing unbound reporter molecules from said first and second solid phase;
h) determining the amount and/or activity of said reporter molecule bound to said first binding compound via said first peptide tag, if present, wherein the amount and/or activity of said reporter molecule linked to said first tag is proportional to the amount and/or activity of said first endogenous compound of said cell, and
i) determining the amount and/or activity of said reporter molecule, if present, bound to said second binding compound via said peptide that is prone to one or more post-translational modifications or via said second peptide tag, wherein the amount and/or activity of said reporter molecule is proportional to the extent of post-translational modification of said peptide that is prone to one or more post-translational modifications.

In another aspect of the invention, compositions, kit of parts and vectors are provided for performing a method according to the present invention for determining the amount and/or activity of at least one endogenous compound of a cell and for testing post-translational modification of at least one peptide sequence within said cell. Further provided is therefore a composition or a kit of parts or a vector, comprising:
- a first nucleic acid molecule comprising a first promoter sequence, operably linked to a nucleic acid sequence encoding a reporter molecule linked to a first peptide tag, wherein said first promoter sequence has an activity that is inducible by a first endogenous compound of said cell; and
- a second nucleic acid molecule comprising a promoter sequence, operably linked to a nucleic acid sequence encoding a reporter molecule linked to a peptide that is prone to one or more post-translational modifications, said nucleic acid sequence optionally also encoding a second peptide tag, wherein said second peptide tag, if present, is different from said first peptide tag.

Said composition or kit of parts or vector preferably also comprises a third nucleic acid molecule as described herein before. In one embodiment, said third nucleic acid molecule comprises a promoter sequence, operably linked to a nucleic acid sequence encoding a reporter molecule linked to a second peptide that is prone to one or more post-translational modifications, said nucleic acid sequence optionally also encoding a third peptide tag, wherein said second peptide that is prone to one or more post-translational modifications is different from said first peptide that is prone to one or more post-translational modifications and wherein said third peptide tag, if present, is different from said first and second peptide tags, so that the amount and/or activity of at least one endogenous compound of a cell and post-translational modifications of at least two peptide sequences within said cell can be determined with a method according to the invention.

In one embodiment said third nucleic acid molecule comprises a promoter sequence, operably linked to a nucleic acid sequence encoding a reporter molecule linked to a third peptide tag, wherein said promoter sequence has an activity that is inducible by a second endogenous compound of said cell, wherein said second endogenous compound is different from said first endogenous compound and wherein said third peptide tag is different from said first and second peptide tags, so that the amount and/or activity of at least two endogenous compounds of a cell and post-translational modification(s) of at least one peptide sequence within said cell can be determined with a method according to the invention.

In another embodiment, the post-translational modification of a peptide sequence and the activity of a protease within one cell is determined simultaneously using a method according to the present invention, using a first nucleic acid molecule comprising a promoter sequence, preferably a constitutively active promoter, operably linked to a nucleic acid sequence encoding a reporter molecule linked to a peptide that is prone to one or more post-translational modifications, said nucleic acid sequence optionally also encoding a first peptide tag, and a second nucleic acid molecule comprising a promoter sequence, preferably a constitutively active promoter, operably linked to a nucleic acid sequence encoding a reporter molecule which is linked to a second peptide tag via a peptide sequence that is a target for degradation by a protease, wherein said first peptide tag, if present, is different from said second peptide tag. Using a method according to the present invention, both nucleic acid molecules are introduced into a cell and after performing the reaction steps according to the present invention the reporter signal, if present, is measured at a first and second solid support. In this particular example, the reporter signal present at said first solid support is proportional to the post-translational modification of said peptide sequence, whereas the reporter signal present at said second solid support is inversely proportional to the activity of said protease.

Further provided is therefore a method for determining post-translational modification of at least one peptide sequence within a cell and the activity of at least one protease in said cell, the method comprising:
a) introducing into said cell a first nucleic acid molecule comprising a promoter sequence, preferably a constitutively active promoter sequence, operably linked to a nucleic acid sequence encoding a reporter molecule linked to a peptide that is prone to one or more post-translational modifications, said nucleic acid sequence optionally also encoding a first peptide tag;
b) introducing into said cell a second nucleic acid molecule that comprises a promoter sequence, preferably a constitutively active promoter, operably linked to a nucleic acid sequence encoding a reporter molecule which is linked to a second peptide tag via a peptide sequence that is a target for degradation by a protease, wherein said first peptide tag, if present, is different from said second peptide tag;
c) culturing said cell, and optionally providing at least one stimulus of interest to said cell;
d) lysing said cell, thereby producing a cell lysate;
e) contacting at least part of said cell lysate with a first binding compound that is bound to, or present at, a first solid phase, wherein said first binding compound is capable of specifically binding said peptide that is prone to one or more post-translational modifications, or said first peptide tag;
f) contacting at least part of said cell lysate with a second binding compound that is bound to, or present at, a second solid phase, wherein said second binding compound is capable of specifically binding said second peptide tag;
g) removing unbound reporter molecules from said first and second solid phase;
h) determining the amount and/or activity of said reporter molecule, if present, bound to said first binding compound via said peptide that is prone to one or more post translational modifications, or via said first peptide tag, wherein the amount and/or activity of said reporter molecule is proportional to the extent of post translational modification of said peptide that is prone to one or more post translational modifications, and
i) determining the amount and/or activity of said reporter molecule, if present, bound to said second binding compound via said second peptide tag, wherein the amount and/or activity of said reporter molecule is inversely proportional to the activity of said second protease.

In another aspect of the invention, compositions, kit of parts and vectors are provided for performing a method according to the present invention for determining post-translational modification of at least one peptide sequence within a cell and the activity of at least one protease in said cell. Further provided is therefore a composition or a kit of parts or a vector, comprising:
- a first nucleic acid molecule comprising a promoter sequence, preferably a constitutively active promoter sequence, operably linked to a nucleic acid sequence encoding a reporter molecule linked to a first peptide that is prone to one or more post-translational modifications, said nucleic acid sequence optionally also encoding a first peptide tag; and
- a second nucleic acid molecule that comprises a promoter sequence, preferably a constitutively active promoter, operably linked to a nucleic acid sequence encoding a reporter molecule which is linked to a second peptide tag via a peptide sequence that is a target for degradation by a protease, wherein said first peptide tag, if present, is different from said second peptide tag.

Said composition or kit of parts or vector preferably also comprises a third nucleic acid molecule as described herein before. In one embodiment said third nucleic acid molecule comprises a promoter sequence, preferably a constitutively active promoter, operably linked to a nucleic acid sequence encoding a reporter molecule which is linked to a third peptide tag via a second peptide sequence that is a target for degradation by a second protease, wherein said second peptide sequence that is a target for degradation by a second protease is different from said first peptide sequence that is a target for degradation by a first protease, and wherein said third peptide tag is different from said first and second peptide tags, so that post-translational modification(s) of at least one peptide sequence within a cell and the activity of at least two different proteases in said cell can be determined with a method according to the invention.

In one embodiment, said third nucleic acid molecule comprises a promoter sequence, operably linked to a nucleic acid sequence encoding a reporter molecule linked to a second peptide that is prone to one or more post-translational modifications, said nucleic acid sequence optionally also encoding a third peptide tag, wherein said second peptide that is prone to one or more post-translational modifications is different from said first peptide that is prone to one or more post-translational modifications and wherein said third peptide tag, if present, is different from said first and second peptide tags, so that post-translational modifications of at least two peptide sequences within a cell and the activity of at least one protease in said cell can be determined with a method according to the invention.

In yet another embodiment, the amount and/or activity of a protein involved in a signal transduction pathway in a cell, and the post-translational modification of a peptide sequence within said cell, and the activity of a protease within said cell is determined simultaneously using a method according to the present invention. In this embodiment, a first nucleic acid molecule is used that comprises a first promoter sequence, operably linked to a nucleic acid sequence encoding a reporter molecule linked to a first peptide tag, wherein said first promoter sequence has an activity that is inducible by said protein involved in a signal transduction pathway, and a second nucleic acid molecule is used that comprises a promoter sequence, preferably a constitutively active promoter, operably linked to a nucleic acid sequence encoding a reporter molecule linked to a peptide that is prone to one or more post-translational modifications, said nucleic acid sequence optionally also encoding a second peptide tag, and a third nucleic acid molecule is used that comprises a promoter sequence, preferably a constitutively active promoter, operably linked to a nucleic acid sequence encoding a reporter molecule which is linked to a third peptide tag via a peptide sequence that is a target for degradation by a protease, wherein said third peptide tag is different from said first peptide tag. If said second peptide tag is present, it is also different from said first and third peptide tags. Using a method according to the present invention, said three nucleic acid molecules are introduced into a cell and after performing the reaction steps according to the present invention the reporter signal, if any, is measured at a first, second and third solid support. In this particular example, the reporter signal present at said first solid support is proportional to the amount and/or activity of said protein involved in a signal transduction pathway, the reporter signal present at said second solid support is proportional to the post-translational modification of said peptide sequence, and the reporter signal present at said third solid support is inversely proportional to the activity of said protease.

Further provided is therefore a method for determining the amount and/or activity of a protein involved in a signal transduction pathway in a cell and the post-translational modification of a peptide sequence within said cell and the activity of a protease within said cell, the method comprising:
a) introducing into said cell a first nucleic acid molecule comprising a first promoter sequence, operably linked to a nucleic acid sequence encoding a reporter molecule linked to a first peptide tag, wherein said first promoter sequence has an activity that is inducible by a first endogenous compound of said cell;
b) introducing into said cell a second nucleic acid molecule comprising a promoter sequence, operably linked to a nucleic acid sequence encoding a reporter molecule linked to a peptide that is prone to one or more post-translational modifications, said nucleic acid sequence optionally also encoding a second peptide tag, wherein said second peptide tag, if present, is different from said first peptide tag;
c) introducing into said cell a third nucleic acid molecule comprising a promoter sequence, preferably a constitutively active promoter, operably linked to a nucleic acid sequence encoding a reporter molecule which is linked to a third peptide tag via a peptide sequence that is a target for degradation by a protease, wherein said third peptide tag is different from said first and second peptide tags;
d) culturing said cell, and optionally providing at least one stimulus of interest to said cell;
e) lysing said cell, thereby producing a cell lysate;
f) contacting at least part of said cell lysate with a first binding compound that is bound to, or present at, a first solid phase, wherein said first binding compound is capable of specifically binding said first peptide tag;
g) contacting at least part of said cell lysate with a second binding compound that is bound to, or present at, a second solid phase, wherein said second binding compound is capable of specifically binding said peptide that is prone to one or more post-translational modifications, or said second peptide tag;
h) contacting at least part of said cell lysate with a third binding compound that is bound to, or present at, a third solid phase, wherein said third binding compound is capable of specifically binding said third peptide tag;
i) removing unbound reporter molecules from said first, second and third solid phases;
j) determining the amount and/or activity of said reporter molecule, if present, bound to said first binding compound via said first peptide tag, wherein the amount and/or activity of said reporter molecule is proportional to the amount and/or activity of said first endogenous compound of said cell;
k) determining the amount and/or activity of said reporter molecule, if present, bound to said second binding compound via said peptide that is prone to one or more post-translational modifications or via said second peptide tag, wherein the amount and/or activity of said reporter molecule is proportional to the extent of post-translational modification of said peptide that is prone to one or more post-translational modifications; and
l) determining the amount and/or activity of said reporter molecule, if present, bound to said third binding compound, wherein the amount and/or activity of said reporter molecule is inversely proportional to the activity of said protease.

In another aspect of the invention, compositions, kit of parts and vectors are provided for performing a method according to the present invention for determining the amount and/or activity of a protein involved in a signal transduction pathway in a cell and the post-translational modification of a peptide sequence within said cell and the activity of a protease within said cell. Further provided is therefore a composition or a kit of parts or a vector, comprising:
- a first nucleic acid molecule comprising a first promoter sequence, operably linked to a nucleic acid sequence encoding a reporter molecule linked to a first peptide tag, wherein said first promoter sequence has an activity that is inducible by a first endogenous compound of said cell; and
- a second nucleic acid molecule comprising a promoter sequence, operably linked to a nucleic acid sequence encoding a reporter molecule linked to a peptide that is prone to one or more post-translational modifications, said nucleic acid sequence optionally also encoding a second peptide tag, wherein said second peptide tag, if present, is different from said first peptide tag; and
- a third nucleic acid molecule comprising a promoter sequence, preferably a constitutively active promoter, operably linked to a nucleic acid sequence encoding a reporter molecule which is linked to a third peptide tag via a peptide sequence that is a target for degradation by a protease, wherein said third peptide tag is different from said first and second peptide tags.

The binding compounds used in the methods according to the present invention can be any compound capable of specifically binding a peptide sequence. "Specific binding" means that the binding compound has an affinity for its target that is higher than overall background affinity. Non-specific sticking is thus not encompassed by this term. In a particularly preferred embodiment, said binding compound is an antibody, or a functional part or functional equivalent thereof. A functional part of an antibody is defined as a part which has at least one same property as said antibody in kind, not necessarily in amount. Said functional part is capable of binding the same antigen as said antibody, albeit not necessarily to the same extent. A functional part of an antibody preferably comprises a single domain antibody, a single chain antibody, a single chain variable fragment (scFv), a diabody, a Fab fragment or a F(ab')₂ fragment.

A Fab fragment is defined as the part of an antibody that contains one constant domain (CH1) and one variable domain of an immunoglobulin heavy chain and one constant domain (CL) and one variable domain of an immunoglobulin light chain.

A F(ab')₂ fragment contains two Fab fragments, joined by disulfide bonds in the hinge region.

A single-chain variable fragment (scFv) is a fusion protein comprising the variable domain of an immunoglobulin heavy chain and the variable domain of an immunoglobulin light chain. These variable domains are typically linked to each other via a linker, preferably a peptide linker. Such peptide linker typically has a length of between 10 and 25 amino acid residues. Linking of two scFvs results in a diabody.

A single-domain antibody (sdAb, also called a nanobody) is an antibody fragment consisting of a single variable region.

A functional equivalent of an antibody is defined as an antibody which has been altered such that at least one property - preferably an antigen-binding property - of the resulting compound is essentially the same in kind, not necessarily in amount. A functional equivalent is provided in many ways, for instance through conservative amino acid substitution, whereby an amino acid residue is substituted by another residue with generally similar properties (size, hydrophobicity, etc), such that the overall functioning is not seriously affected. Also fusion proteins and chimeric antibodies are encompassed by the term functional equivalent.

As stated herein before, it is preferred to perform a method according to the present invention with at least two different nucleic acid molecules which contain the same reporter gene. Preferably, at least three different nucleic acid molecules contain the same reporter gene. In a particularly preferred embodiment, all nucleic acid molecules used in a method according to the invention contain the same reporter gene. The use of the same reporter gene is preferred since it facilitates production efficacy and it provides the possibility to use one preferred reporter which is for instance very sensitive, or readily available. Further provided is therefore a method, composition, kit of parts or vector according to the invention, wherein said first and said second nucleic acid molecules, and preferably also a third nucleic acid molecule, most preferably all nucleic acid molecules, comprise a nucleic acid sequence encoding the same reporter molecule.

In one embodiment, said reporter molecule comprises an enzyme that is capable of catalyzing a light generating reaction. This facilitates detection of a reporter signal. In a particularly preferred embodiment, said reporter molecule comprises a luciferase.

In one preferred embodiment, a method, composition, kit of parts or vector according the invention is provided, wherein at least one of said nucleic acid molecules further comprises a sequence encoding a marker protein which, when expressed by a cell, allows said cell to survive an otherwise toxic stimulus. This way, transfected cells are selected, since untransfected cells will not survive the toxic stimulus.

A composition or kit of parts according to the invention preferably further comprises a binding compound that is capable of specifically binding said first peptide tag and a binding compound capable of specifically binding said second peptide tag, in order to allow detection of reporter signals at a solid phase. Most preferably, a composition or kit of parts according to the invention further comprises suitable means for detecting expression of said reporter molecule.

The invention furthermore provides a cell comprising a vector according to the invention. Such cell thus comprises a combination of at least two nucleic acid molecules according to the invention. Such cell is particular suitable for performing the methods according to the invention, for instance for investigating multiple cellular pathways or processes, post-translational modifications, and/or the activity of multiple endogenous compounds such as transcription factors, proteases and compounds involved in signal transduction.

The invention also provides uses of combinations of nucleic acid molecules according to the invention for performing any of the methods provided herewith.

Further provided is therefore a use of:
- a first nucleic acid molecule comprising a first promoter sequence, operably linked to a nucleic acid sequence encoding a reporter molecule linked to a first peptide tag, wherein said first promoter sequence has an activity that is inducible by a first cellular endogenous compound; and
- a second nucleic acid molecule comprising a second promoter sequence, operably linked to a nucleic acid sequence encoding a reporter molecule linked to a second peptide tag, wherein said second promoter sequence has an activity that is inducible by a second cellular endogenous compound, wherein said first peptide tag is different from said second peptide tag,
for determining the amount and/or activity of at least two endogenous compounds of a cell.

Also provided is a use of:
- a first nucleic acid molecule comprising a promoter sequence, operably linked to a nucleic acid sequence encoding a reporter molecule linked to a first peptide that is prone to one or more post-translational modifications, said nucleic acid sequence optionally also encoding a first peptide tag; and
- a second nucleic acid molecule comprising a promoter sequence, operably linked to a nucleic acid sequence encoding a reporter molecule linked to a second peptide that is prone to one or more post-translational modifications, said nucleic acid sequence optionally also encoding a second peptide tag, wherein said first peptide that is prone to one or more post-translational modifications is different from said second peptide that is prone to one or more post-translational modifications, and, optionally, wherein said first peptide tag is different from said second peptide tag;
for testing post-translational modifications of at least two peptide sequences within one cell.

Also provided is a use of:
- a first nucleic acid molecule that comprises a promoter sequence, preferably a constitutively active promoter, operably linked to a nucleic acid sequence encoding a reporter molecule which is linked to a first peptide tag via a first peptide sequence that is a target for degradation by a first protease; and
- a second nucleic acid molecule that comprises a promoter sequence, preferably a constitutively active promoter, operably linked to a nucleic acid sequence encoding a reporter molecule which is linked to a second peptide tag via a second peptide sequence that is a target for degradation by a second protease, wherein said first peptide sequence that is a target for degradation by a protease is different from said second peptide sequence that is a target for degradation by a protease, and wherein said first peptide tag is different from said second peptide tag;
for determining the activity of at least two different proteases in a cell.

Another embodiment provides a use of:
- a first nucleic acid molecule comprising a first promoter sequence, operably linked to a nucleic acid sequence encoding a reporter molecule linked to a first peptide tag, wherein said first promoter sequence has an activity that is inducible by a first endogenous compound of a cell; and
- a second nucleic acid molecule that comprises a promoter sequence, preferably a constitutively active promoter, operably linked to a nucleic acid sequence encoding a reporter molecule which is linked to a second peptide tag via a peptide sequence that is a target for degradation by a protease, wherein said first peptide tag is different from said second peptide tag;
for determining the amount and/or activity of at least one endogenous compound of a cell and the activity of at least one protease in said cell.

Further provided is a use of:
- a first nucleic acid molecule comprising a first promoter sequence, operably linked to a nucleic acid sequence encoding a reporter molecule linked to a first peptide tag, wherein said first promoter sequence has an activity that is inducible by a first endogenous compound of a cell; and
- a second nucleic acid molecule comprising a promoter sequence, operably linked to a nucleic acid sequence encoding a reporter molecule linked to a second peptide that is prone to one or more post-translational modifications, said nucleic acid sequence optionally also encoding a second peptide tag, wherein said second peptide tag, if present, is different from said first peptide tag
for determining the amount and/or activity of at least one endogenous compound of a cell and for testing post-translational modification of at least one peptide sequence within said cell.

Also provided is a use of:
- a first nucleic acid molecule comprising a promoter sequence, operably linked to a nucleic acid sequence encoding a reporter molecule linked to a peptide that is prone to one or more post-translational modifications, said nucleic acid sequence optionally also encoding a first peptide tag; and
- a second nucleic acid molecule that comprises a promoter sequence, preferably a constitutively active promoter, operably linked to a nucleic acid sequence encoding a reporter molecule which is linked to a second peptide tag via a peptide sequence that is a target for degradation by a protease, wherein said first peptide tag, if present, is different from said second peptide tag;
for determining post-translational modification of at least one peptide sequence within a cell and the activity of at least one protease in a cell.

Also provided is a use of:
- a first nucleic acid molecule comprising a first promoter sequence, operably linked to a nucleic acid sequence encoding a reporter molecule linked to a first peptide tag, wherein said first promoter sequence has an activity that is inducible by a first endogenous compound of said cell; and
- a second nucleic acid molecule comprising a promoter sequence, operably linked to a nucleic acid sequence encoding a reporter molecule linked to a peptide that is prone to one or more post-translational modifications, said nucleic acid sequence optionally also encoding a second peptide tag, wherein said second peptide tag, if present, is different from said first peptide tag; and
- a third nucleic acid molecule comprising a promoter sequence, preferably a constitutively active promoter, operably linked to a nucleic acid sequence encoding a reporter molecule which is linked to a third peptide tag via a peptide sequence that is a target for degradation by a protease, wherein said third peptide tag is different from said first and second peptide tags;
for determining the amount and/or activity of a protein involved in a signal transduction pathway in a cell and the post-translational modification of a peptide sequence within said cell and the activity of a protease within said cell.

A use of a composition or kit of parts according to the invention for following at least one cellular process or pathway is also provided herewith. Said pathway is preferably a Wnt pathway.

In one embodiment, a method according to the invention is provided wherein multiple nucleic acid molecules according to the invention are introduced into a cell mixture instead of single cells. This provides the advantage that even more different nucleic acid molecules can be introduced into the cell culture as a whole, going beyond the insertion capacity of a single cell. Preferably, only one cell culture is used, containing a mixture of cells, because then all cells have the same reaction conditions, intercellular interactions can take place, and the use of expensive materials, or materials with limited availability such as materials derived from biological samples, is minimized.

### Brief description of the drawings

Figure 1: schematic, non-limited overview of one embodiment of a method according to the present invention, as compared to a conventional method.

## Claims

1. Method for determining the amount and/or activity of at least two endogenous compounds of a cell, the method comprising:
a) introducing into said cell a first nucleic acid molecule comprising a first promoter sequence, operably linked to a nucleic acid sequence encoding a reporter molecule linked to a first peptide tag, wherein said first promoter sequence has an activity that is inducible by a first endogenous compound of said cell;
b) introducing into said cell a second nucleic acid molecule comprising a second promoter sequence, operably linked to a nucleic acid sequence encoding a reporter molecule linked to a second peptide tag, wherein said second promoter sequence has an activity that is inducible by a second endogenous compound of said cell, and wherein said first peptide tag is different from said second peptide tag;
c) culturing said cell, and optionally providing at least one stimulus of interest to said cell;
d) lysing said cell, thereby producing a cell lysate;
e) contacting at least part of said cell lysate with a first binding compound that is bound to, or present at, a first solid phase, wherein said first binding compound is capable of specifically binding said first peptide tag;
f) contacting at least part of said cell lysate with a second binding compound that is bound to, or present at, a second solid phase, wherein said second binding compound is capable of specifically binding said second peptide tag;
g) removing unbound reporter molecules from said first and second solid phase;
h) determining the amount and/or activity of said reporter molecule bound to said first binding compound via said first peptide tag, wherein the amount and/or activity of said reporter molecule linked to said first tag is indicative for the amount and/or activity of said first endogenous compound of said cell, and
i) determining the amount and/or activity of said reporter molecule bound to said second binding compound via said second peptide tag, wherein the amount and/or activity of said reporter molecule linked to said second tag is indicative for the amount and/or activity of said second endogenous compound of said cell.

2. A method according to claim 1, further comprising:
- introducing into said cell a third nucleic acid molecule comprising a third promoter sequence, operably linked to a nucleic acid sequence encoding a reporter molecule linked to a third peptide tag, wherein said third promoter sequence has an activity that is inducible by a third endogenous compound of said cell, wherein said third promoter sequence is different from said first and second promoter sequences and wherein said third peptide tag is different from said first and second peptide tag;
- contacting at least part of said cell lysate with a third binding compound that is bound to, or present at, a third solid phase, wherein said third binding compound is capable of specifically binding said third peptide tag;
- removing unbound reporter molecules from said third solid phase; and
- determining the amount and/or activity of said reporter molecule bound to said third binding compound via said third peptide tag, wherein the amount and/or activity of said third reporter molecule is indicative for the amount and/or activity of said third endogenous compound of said cell.

3. A composition or a kit of parts or a vector, comprising:
- a first nucleic acid molecule comprising a first promoter sequence, operably linked to a nucleic acid sequence encoding a reporter molecule linked to a first peptide tag, wherein said first promoter sequence has an activity that is inducible by a first cellular endogenous compound; and
- a second nucleic acid molecule comprising a second promoter sequence, operably linked to a nucleic acid sequence encoding a reporter molecule linked to a second peptide tag, wherein said second promoter sequence has an activity that is inducible by a second cellular endogenous compound, wherein said first promoter sequence is different from said second promoter sequence and wherein said first peptide tag is different from said second peptide tag.

4. A method for testing post-translational modifications of at least two peptide sequences within one cell, the method comprising:
a) introducing into said cell a first nucleic acid molecule comprising a promoter sequence, operably linked to a nucleic acid sequence encoding a reporter molecule linked to a first peptide that is prone to one or more post-translational modifications, said nucleic acid sequence optionally also encoding a first peptide tag;
b) introducing into said cell a second nucleic acid molecule comprising a promoter sequence, operably linked to a nucleic acid sequence encoding a reporter molecule linked to a second peptide that is prone to one or more post-translational modifications, said nucleic acid sequence optionally also encoding a second peptide tag, wherein said first peptide that is prone to one or more post-translational modifications is different from said second peptide that is prone to one or more post-translational modifications, and, optionally, wherein said first peptide tag is different from said second peptide tag;
c) culturing said cell, and optionally providing at least one stimulus of interest to said cell;
d) lysing said cell, thereby producing a cell lysate;
e) contacting at least part of said cell lysate with a first binding compound that is bound to, or present at, a first solid phase, wherein said first binding compound is capable of specifically binding said first peptide that is prone to one or more post-translational modifications, or said first peptide tag;
f) contacting at least part of said cell lysate with a second binding compound that is bound to, or present at, a second solid phase, wherein said second binding compound is capable of specifically binding said second peptide that is prone to one or more post-translational modifications, or said second peptide tag;
g) removing unbound reporter molecules from said first and second solid phase;
h) determining the amount and/or activity of said reporter molecule bound to said first binding compound via said first peptide that is prone to one or more post translational modifications, or via said first peptide tag, wherein the amount and/or activity of said reporter molecule is proportional to the extent of post translational modification of said first peptide that is prone to one or more post translational modifications, and
i) determining the amount and/or activity of said reporter molecule bound to said second binding compound via said second peptide that is prone to one or more post-translational modifications or via said second peptide tag, wherein the amount and/or activity of said reporter molecule is proportional to the extent of post-translational modification of said second peptide that is prone to one or more post-translational modifications.

5. A composition or a kit of parts or a vector, comprising:
- a first nucleic acid molecule comprising a promoter sequence, operably linked to a nucleic acid sequence encoding a reporter molecule linked to a first peptide that is prone to one or more post-translational modifications, said nucleic acid sequence optionally also encoding a first peptide tag; and
- a second nucleic acid molecule comprising a promoter sequence, operably linked to a nucleic acid sequence encoding a reporter molecule linked to a second peptide that is prone to one or more post-translational modifications, said nucleic acid sequence optionally also encoding a second peptide tag, wherein said first peptide that is prone to one or more post-translational modifications is different from said second peptide that is prone to one or more post-translational modifications, and, optionally, wherein said first peptide tag is different from said second peptide tag.

6. A method for determining the activity of at least two different proteases in a cell, the method comprising:
a) introducing into said cell a first nucleic acid molecule that comprises a promoter sequence, preferably a constitutively active promoter, operably linked to a nucleic acid sequence encoding a reporter molecule which is linked to a first peptide tag via a first peptide sequence that is a target for degradation by a first protease;
b) introducing into said cell a second nucleic acid molecule that comprises a promoter sequence, preferably a constitutively active promoter, operably linked to a nucleic acid sequence encoding a reporter molecule which is linked to a second peptide tag via a second peptide sequence that is a target for degradation by a second protease, wherein said first peptide sequence that is a target for degradation by a protease is different from said second peptide sequence that is a target for degradation by a protease, and wherein said first peptide tag is different from said second peptide tag;
c) culturing said cell, and optionally providing at least one stimulus of interest to said cell;
d) lysing said cell, thereby producing a cell lysate;
e) contacting at least part of said cell lysate with a first binding compound that is bound to, or present at, a first solid phase, wherein said first binding compound is capable of specifically binding said first peptide tag;
f) contacting at least part of said cell lysate with a second binding compound that is bound to, or present at, a second solid phase, wherein said second binding compound is capable of specifically binding said second peptide tag;
g) removing unbound reporter molecules from said first and second solid phase;
h) determining the amount and/or activity of reporter molecule bound to said first binding compound via said first peptide tag, if present, wherein the amount and/or activity of said reporter molecule is inversely proportional to the activity of said first protease, and
i) determining the amount and/or activity of reporter molecule bound to said second binding compound via said second peptide tag, if present, wherein the amount and/or activity of said reporter molecule is inversely proportional to the activity of said second protease.

7. A composition or a kit of parts or a vector, comprising:
- a first nucleic acid molecule that comprises a promoter sequence, preferably a constitutively active promoter, operably linked to a nucleic acid sequence encoding a reporter molecule which is linked to a first peptide tag via a first peptide sequence that is a target for degradation by a first protease; and
- a second nucleic acid molecule that comprises a promoter sequence, preferably a constitutively active promoter, operably linked to a nucleic acid sequence encoding a reporter molecule which is linked to a second peptide tag via a second peptide sequence that is a target for degradation by a second protease, wherein said first peptide sequence that is a target for degradation by a protease is different from said second peptide sequence that is a target for degradation by a protease, and wherein said first peptide tag is different from said second peptide tag.

8. A method, composition, kit of parts or vector according to any one of claims 1-7, wherein said binding compound is an antibody, or a functional part or functional equivalent thereof.

9. A method, composition, kit of parts or vector according to any one of claims 1-8, wherein said first and said second nucleic acid molecules comprise a nucleic acid sequence encoding the same reporter molecule.

10. A method, composition, kit of parts or vector according to any one of claims 1-9, wherein said reporter molecule comprises an enzyme that is capable of catalyzing a light generating reaction.

11. A method, composition, kit of parts or vector according to any one of claims 1-10, wherein said reporter molecule comprises a luciferase.

12. A method, composition, kit of parts or vector according to any one of claims 1-11, wherein said first promoter sequence and/or said second promoter sequence comprises a transcription factor binding site.

13. A method, composition, kit of parts or vector according to any one of claims 1-12, wherein said first promoter sequence and/or said second promoter sequence is operably linked to a transcription factor binding site.

14. A method, composition, kit of parts or vector according to any one of claims 1-13, wherein said endogenous compounds are involved in a cellular process, preferably a signal transduction pathway.

15. A method, composition, kit of parts or vector according to any one of claims 1-14, wherein the activity of said first promoter sequence and/or the activity of said second promoter sequence is inducible by a Wnt factor.

16. A method, composition, kit of parts or vector according to any one of claims 1-15, wherein said nucleic acid molecule further comprises a sequence encoding a marker protein which, when expressed by a cell, allows said cell to survive an otherwise toxic stimulus.

17. A cell comprising a vector according to any one of claims 3-16.

18. A composition or kit of parts according to any one of claims 3-17, further comprising a binding compound that is capable of specifically binding to said first peptide tag and a binding compound capable of specifically binding to said second peptide tag.

19. Use of:
- a first nucleic acid molecule comprising a first promoter sequence, operably linked to a nucleic acid sequence encoding a reporter molecule linked to a first peptide tag, wherein said first promoter sequence has an activity that is inducible by a first cellular endogenous compound; and
- a second nucleic acid molecule comprising a second promoter sequence, operably linked to a nucleic acid sequence encoding a reporter molecule linked to a second peptide tag, wherein said second promoter sequence has an activity that is inducible by a second cellular endogenous compound, wherein said first promoter sequence is different from said second promoter sequence and wherein said first peptide tag is different from said second peptide tag,
for determining the amount and/or activity of at least two endogenous compounds of a cell.

20. Use of:
- a first nucleic acid molecule comprising a promoter sequence, operably linked to a nucleic acid sequence encoding a reporter molecule linked to a first peptide that is prone to one or more post-translational modifications, said nucleic acid sequence optionally also encoding a first peptide tag; and
- a second nucleic acid molecule comprising a promoter sequence, operably linked to a nucleic acid sequence encoding a reporter molecule linked to a second peptide that is prone to one or more post-translational modifications, said nucleic acid sequence optionally also encoding a second peptide tag, wherein said first peptide that is prone to one or more post-translational modifications is different from said second peptide that is prone to one or more post-translational modifications, and, optionally, wherein said first peptide tag is different from said second peptide tag;
for testing post-translational modifications of at least two peptide sequences within one cell.

21. Use of:
- a first nucleic acid molecule that comprises a promoter sequence, preferably a constitutively active promoter, operably linked to a nucleic acid sequence encoding a reporter molecule which is linked to a first peptide tag via a first peptide sequence that is a target for degradation by a first protease; and
- a second nucleic acid molecule that comprises a promoter sequence, preferably a constitutively active promoter, operably linked to a nucleic acid sequence encoding a reporter molecule which is linked to a second peptide tag via a second peptide sequence that is a target for degradation by a second protease, wherein said first peptide sequence that is a target for degradation by a protease is different from said second peptide sequence that is a target for degradation by a protease, and wherein said first peptide tag is different from said second peptide tag;
for determining the activity of at least two different proteases in a cell.

22. Use of a composition or kit of parts according to any one of claims 3-18, for following at least one cellular process or pathway.

23. Use according to claim 22, wherein said pathway is a Wnt pathway.
